# EUROPEAN PATENT APPLICATION

(11) **EP 4 793 897 A1**
(43) Date of publication of application: **19.08.2026**
(21) Application number: 26156059.3
(22) Date of filing: 03.02.2026
(51) Int. Cl.: G06V 10/26, G06T 7/11, G06V 10/80, G06V 10/82, G16H 20/00, G16H 30/20, G16H 30/40, G16H 50/20, G16H 50/70

(54) **MEDICAL IMAGE SEMANTIC SEGMENTATION METHOD AND APPARATUS, ELECTRONIC DEVICE, AND STORAGE MEDIUM**

(30) Priority: 14.02.2025 CN 202510163039
(71) Applicant: Peking Union Medical College Hospital, Chinese Academy of Medical Sciences & Peking Union Medical College, Beijing 100730 (CN); Digital Health China Technologies Co., Ltd., Beijing 100080 (CN)
(72) Inventor: ZHANG, Shuyang, Beijing 100730 (CN); GUO, Jian, Beijing 100730 (CN); JIN, Ye, Beijing 100730 (CN); GONG, Mengchun, Beijing 100080 (CN); SHI, Wenzhao, Beijing 100080 (CN); ZHENG, Xihong, Beijing 100080 (CN); LIU, Peng, Beijing 100730 (CN)
(74) Representative: Deambrogi, Edgardo

(57) **Abstract**

The present invention provides a medical image semantic segmentation method and apparatus, an electronic device, and a storage medium. The method includes: extracting, through a feature extraction network, a first image feature corresponding to a first image and multiple second image features corresponding to at least one second image, respectively; performing feature cross-alignment between the first image feature and a mean of the multiple second image features, so as to obtain multiple cross features; performing optimization on the multiple cross features, respectively, based on a similarity between the first image feature and the multiple second image features, so as to acquire multiple optimized features; fusing, through a decoder, the first image feature, the multiple optimized features, and masks matching each optimized feature, so as to obtain a mask result corresponding to the first image.

## Description

### TECHNICAL FIELD

The present invention relates to the field of image processing technologies, and specifically to a medical image semantic segmentation method and apparatus, an electronic device, and a storage medium.

### BACKGROUND ART

With the continuous advancement of medical imaging technology, the image semantic segmentation technology is increasingly widely applied in the field of medical diagnosis. This technology enables automatic identification and segmentation of different tissues and organs in medical images, thereby assisting physicians in more accurately diagnosing diseases and formulating treatment plans. In the prior art, image semantic segmentation algorithms typically rely on large amounts of annotated data to train models, so as to achieve high-precision segmentation effects. However, in the case of rare diseases, the limited cases correspondingly result in a scarcity of available medical image samples, which makes it difficult for existing image semantic segmentation technologies to obtain sufficient training data, thereby affecting the performance thereof in diagnosing rare diseases. Therefore, under the prior art conditions, image semantic segmentation effects are often unsatisfactory when processing rare disease-related medical images.

### SUMMARY

Embodiments of the present invention aim at providing a medical image semantic segmentation method and apparatus, an electronic device, and a storage medium, for improving accuracy of rare disease medical image semantic segmentation.

In the first aspect, the present invention provides a medical image semantic segmentation method, including: extracting, through a feature extraction network, a first image feature corresponding to a first image and multiple second image features corresponding to at least one second image, respectively, where the at least one second image is image(s) of a target patient taken during historical medical visits and having undergone semantic segmentation for a medical semantic label, and the first image is an image of the target patient currently taken and having pixel regions with the medical semantic label; performing feature cross-alignment between the first image feature and a mean of the multiple second image features, so as to obtain multiple cross features; performing optimization on the multiple cross features, respectively, based on a similarity between the first image feature and the multiple second image features, so as to acquire multiple optimized features; fusing, through a decoder, the first image feature, the multiple optimized features, and masks matching each optimized feature, so as to obtain a mask result corresponding to the first image, where the mask result is configured to indicate target pixel regions belonging to the medical semantic label and non-target pixel regions excluded from the medical semantic label in the first image, where a corresponding cross feature for the first image feature and each second image feature is obtained by a method as follows:

calculating a product, for a first element value at each position in the first image feature, between the first element value and a second element value at a corresponding position in the second image feature, as a third element value at a corresponding position in the corresponding cross feature.

In an optional embodiment, the feature extraction network includes a first convolutional block, a second convolutional block, a third convolutional block, a fourth convolutional block, and a fifth convolutional block connected in sequence, where each convolutional block includes a first convolutional layer, an asymmetric convolutional layer, and a second convolutional layer connected in sequence.

In an optional embodiment, for the first image feature and each second image feature, optimization is performed on the corresponding cross feature by a following method, so as to acquire corresponding optimized feature:

determining a first element value distance feature based on the first image feature and a current second image feature; determining a second element value distance feature based on the first image feature and remaining second image features; determining, for the third element value at each position in a current cross feature, whether the third element value at the position requires optimization, based on a first distance value at the corresponding position in the first element value distance feature and a second distance value at the corresponding position in the second element value distance feature; and modifying the third element value, if the third element value at the position requires optimization, based on the second element value at the position in the current second image feature, as a fourth element value at a corresponding position in the corresponding optimized feature.

In an optional embodiment, whether the third element value at each position requires optimization is determined by a method as follows:

determining whether the first distance value corresponding to the position is greater than a first target value and whether the second distance value corresponding to the position is less than a second target value, and if the first distance value is greater than the first target value and the second distance value is less than the second target value, determining that the third element value requires optimization.

In an optional embodiment, the fourth element value is calculated by a method as follows:

multiplying the third element value at corresponding position in the current cross feature by an amplification factor to obtain a product as the fourth element value.

In an optional embodiment, the first element value distance feature is determined by a method as follows:

performing decomposition on the first element value at each position in the first image feature and the second element value at a corresponding position in the current second image feature, so as to acquire a first integer element value and a first decimal element value corresponding to the first element value, and a second integer element value and a second decimal element value corresponding to the second element value, respectively; calculating a first difference between the first integer element value and the second integer element value; calculating a second difference between the first decimal element value and the second decimal element value; and calculating a sum of an absolute value of the first difference and an absolute value of the second difference as a first distance value at a corresponding position in the first element value distance feature.

In an optional embodiment, the second element value distance feature is determined by a method as follows:

calculating, for second element values at corresponding position in the remaining second image features, a second element mean at the position; and calculating a cosine similarity for the first element value at each position in the first image feature and the second element mean at a corresponding position, as a second distance value at corresponding position in the second element value distance feature.

In an optional embodiment, the first convolutional block has a convolutional kernel of 3×3×3, the second convolutional block has a convolutional kernel of 3×1×1, the third convolutional block has a convolutional kernel of 1×3×1, the fourth convolutional block has a convolutional kernel of 1×1×3, and the fifth convolutional block has a convolutional kernel of 1×1×1.

In an optional embodiment, the feature cross-alignment between the first image feature and the mean of the multiple second image features is performed by using Hadamard Product.

In an optional embodiment, Manhattan distance is utilized to calculate a pixel-wise similarity between the first image feature and the second image feature.

In an optional embodiment, the amplification factor ranges between 0 and 1.

In an optional embodiment, the decoder is a TransUnet decoder.

In the second aspect, the present invention provides a medical image semantic segmentation apparatus, including:
an extraction module, configured to extract, through a feature extraction network, a first image feature corresponding to a first image and multiple second image features corresponding to at least one second image, respectively, where the at least one second image is image(s) of a target patient taken during historical medical visits and having undergone semantic segmentation for a medical semantic label, and the first image is an image of the target patient currently taken and having pixel regions with the medical semantic label;
an alignment module, configured to perform feature cross-alignment between the first image feature and a mean of the multiple second image features, so as to obtain multiple cross features, where a corresponding cross feature for the first image feature and each second image feature is obtained by a method as follows:
calculating a product, for a first element value at each position in the first image feature, between the first element value and a second element value at a corresponding position in the second image feature, as a third element value at a corresponding position in the corresponding cross feature;
an optimization module, configured to perform optimization on the multiple cross features, respectively, based on a similarity between the first image feature and the multiple second image features, so as to acquire multiple optimized features; and
a decoding module, configured to fuse, through a decoder, the first image feature, the multiple optimized features, and masks matching each optimized feature, so as to obtain a mask result corresponding to the first image, where the mask result is configured to indicate target pixel regions belonging to the medical semantic label and non-target pixel regions excluded from the medical semantic label in the first image.

In the third aspect, the present invention provides an electronic device, including: a processor, a memory, and a bus, where the memory stores machine-readable instructions executable by the processor, when the electronic device is running, the processor and the memory communicate via the bus, and the processor executes the machine-readable instructions, so as to execute the steps of the medical image semantic segmentation method according to any embodiment in the preceding.

In the fourth aspect, the present invention provides a computer-readable storage medium, where a computer program is stored on the computer-readable storage medium, and the computer program, when run by a processor, executes the steps of the medical image semantic segmentation method according to any embodiment in the preceding.

The medical image semantic segmentation method and apparatus, the electronic device, and the storage medium provided in the present invention take both a segmented image and an unsegmented image of the patient with the same medical semantic label as input to perform feature extraction, perform alignment and optimization on the extracted features, so as to further enhance the descriptive effect of certain features for the medical semantic label, and finally decode to obtain a segmentation result of the medical image of the patient, thus improving the accuracy of the medical semantic segmentation in the rare disease diagnosis and treatment, and providing assistance for diagnosis and treatment of the rare disease patients.

### BRIEF DESCRIPTION OF DRAWINGS

In order to more clearly illustrate technical solutions of embodiments of the present invention, drawings to be used in the embodiments of the present invention will be briefly introduced below. It should be understood that the following drawings only show certain embodiments of the present invention, and thus should not be regarded as limitation to the scope. For those ordinarily skilled in the field, other relevant drawings also could be obtained according to these drawings without making inventive efforts.
FIG. 1 is a flowchart for a medical image semantic segmentation method provided in embodiments of the present invention;
FIG. 2 is a flowchart of steps for feature optimization provided in embodiments of the present invention;
FIG. 3 is a structural schematic diagram of a medical image semantic segmentation apparatus provided in embodiments of the present invention; and
FIG. 4 is a structural schematic diagram of an electronic device provided in embodiments of the present invention.

### DETAILED DESCRIPTION OF EMBODIMENTS

First, an application scenario of the present invention is introduced. Technical solutions of the present invention are applicable to image semantic segmentation in medical diagnosis.

The technical solutions in the embodiments of the present invention will be described below in conjunction with drawings in the embodiments of the present invention.

### Embodiment 1

In the diagnosis and treatment of rare disease patients, due to complexity of rare diseases and individual variability, coupled with the scarcity of case samples, training and constructing models in conventional methods often yield unsatisfactory segmentation effects. Therefore, in an embodiment of the present invention, medical images from the same patient can be employed, which specifically can be MRI, CT, or the like.

Taking a medical semantic label being tumor as an example, MRI images taken during historical visits of the patient are processed to generate corresponding masks.

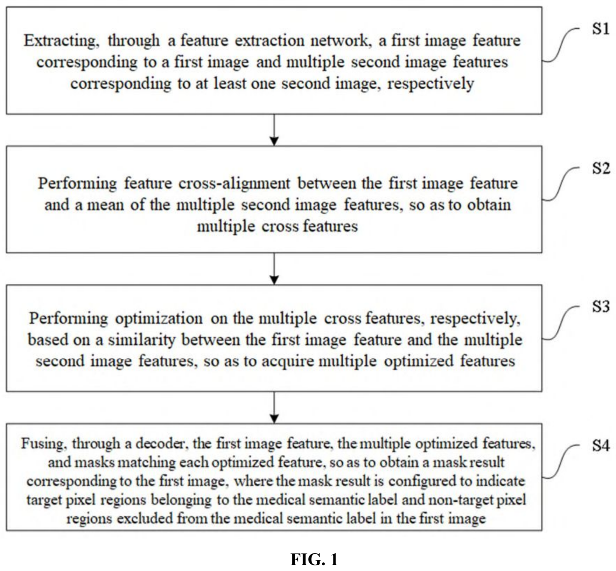

FIG. 1 is a flowchart for a medical image semantic segmentation method provided in embodiments of the present invention. As shown in FIG. 1, embodiments of the present invention provide a medical image semantic segmentation method, executable by a computer device, and including steps as follows.

S1: extracting, through a feature extraction network, a first image feature corresponding to a first image and multiple second image features corresponding to at least one second image, respectively.

Herein, the at least one second image may be brain MRI image(s) of a target patient taken during historical medical visits and having undergone semantic segmentation for the medical semantic label, and the first image is a brain MRI image of the target patient currently taken and having pixel regions with the medical semantic label.

The feature extraction network constructed based on Resnet is employed herein to perform feature extraction on the first image and the at least one second image.

Specifically, the feature extraction network includes a first convolutional block, a second convolutional block, a third convolutional block, a fourth convolutional block, and a fifth convolutional block connected in sequence, wherein each convolutional block includes a first convolutional layer, an asymmetric convolutional layer, and a second convolutional layer connected in sequence. The first convolutional block has a convolutional kernel of 3×3×3, the second convolutional block has a convolutional kernel of 3×1×1, the third convolutional block has a convolutional kernel of 1×3×1, the fourth convolutional block has a convolutional kernel of 1×1×3, and the fifth convolutional block has a convolutional kernel of 1×1×1. Herein, sizes of the first convolutional layer and the second convolutional layer are 3×3×3 and 1×1×1, respectively.

The first image feature and the second image features herein can be features output by the fifth convolutional block. Taking that there are two second images as an example, first image feature *Vₐ* and second image features ${V}_{b}^{1}$ and ${V}_{b}^{2}$ can be obtained, respectively.

Further, in order to improve accuracy of semantic segmentation, features output by the third convolutional block, the fourth convolutional block, and the fifth convolutional block can be extracted as corresponding first image feature or second image features.

S2: performing feature cross-alignment between the first image feature and a mean of the multiple second image features, so as to obtain multiple cross features.

Herein, a corresponding cross feature for the first image feature and each second image feature is obtained by a method as follows:
calculating a product, for a first element value at each position in the first image feature, between the first element value and a second element value at a corresponding position in the second image feature, as a third element value at a corresponding position in the corresponding cross feature.

Taking the first image feature *Vₐ* ∈ *M* × *N* × *R* and the second image feature ${V}_{b}^{1} ∈ M \times N \times R$ as an example, in step S2, Hadamard Product can be employed for performing feature crossing.

A product between a first element value ${v}_{a}^{1 , 1 , 1}$ at (1,1,1) in the first image feature *Vₐ* and a second element value ${v}_{b 1}^{1 , 1 , 1}$ at (1,1,1) in the second image feature ${V}_{b}^{1}$ is calculated as a third element value at (1,1,1) in the cross feature. This process is repeated likewise, ultimately yielding cross feature *K*₁ ∈ *M* × *N* × *R.*

By crossing features between medical images awaiting semantic segmentation and the medical image having undergone the semantic segmentation, a descriptive effect of some features on the medical semantic label can be enhanced.

S3: performing optimization on the multiple cross features, respectively, based on a similarity between the first image feature and the multiple second image features, so as to acquire multiple optimized features.

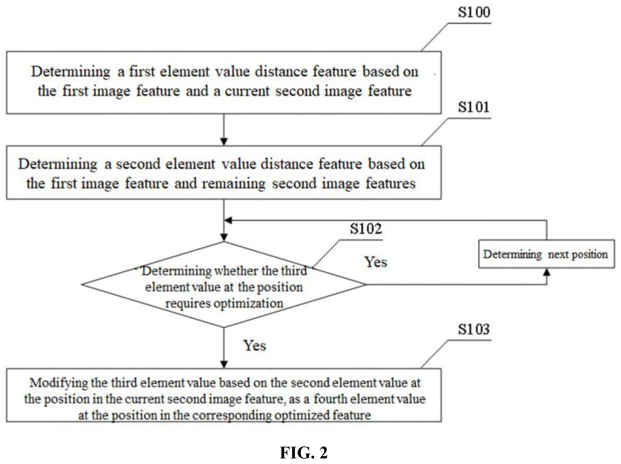

As shown in FIG. 2, taking the first image feature *Vₐ* ∈ *M* × *N* × *R* and the second image feature ${V}_{b}^{1} ∈ M \times N \times R$ as an example, optimization can be performed on the corresponding cross feature by a following method, so as to acquire corresponding optimized feature.

S100: determining a first element value distance feature based on the first image feature and a current second image feature.

In step S100, taking the first image feature *Vₐ* ∈ *M* × *N* × *R* and the second image feature ${V}_{b}^{1} ∈ M \times N \times R$ as an example, a corresponding first element value distance feature can be determined by a method as follows:
performing decomposition on the first element value at each position in the first image feature and the second element value at a corresponding position in the current second image feature, so as to acquire a first integer element value and a first decimal element value corresponding to the first element value, and a second integer element value and a second decimal element value corresponding to the second element value, respectively;
decomposing the first element value ${v}_{a}^{1 , 1 , 1} = 1.36$ at (1,1,1) in the first image feature *Vₐ* into an integer part as the first integer element value 1 and a decimal part as the first decimal element value 0.36;
decomposing the second element value ${v}_{b 1}^{1 , 1 , 1} = 0.51$ at (1,1,1) in the second image feature ${V}_{b}^{1}$ into an integer part as the second integer element value 0 and a decimal part as the second decimal element value 0.51;
calculating a first difference between the first integer element value and the second integer element value; calculating a second difference between the first decimal element value and the second decimal element value; calculating a sum of an absolute value of the first difference and an absolute value of the second difference as a first distance value at a corresponding position in the first element value distance feature; and
calculating the first distance value ${v}_{D 1}^{1 , 1 , 1} = \left|1-0\right| + \left|0.36-0.51\right| = 1.15$ at (1,1,1) in the first element value distance feature. This process is repeated likewise, yielding *D*₁ ∈ *M* × *N* × *R.*

Herein, Manhattan distance is utilized to calculate a pixel-wise similarity between the first image feature and the second image feature, which can reduce sensitivity to abnormal values and better capture similarity between pixels.

S101: determining a second element value distance feature based on the first image feature and remaining second image features.

Taking the first image feature *Vₐ* ∈ *M* × *N* × *R*, and the second image feature ${V}_{b}^{2} ∈ M \times N \times R$ and ${V}_{b}^{3}$ as an example, corresponding second element value distance features can be determined by a method as follows:
for second element values at corresponding position in the remaining second image features, calculating a second element mean at the position:
calculating a mean between second element values ${v}_{b 2}^{1 , 1 , 1}$ and *c* at (1,1,1) in ${V}_{b}^{2}$ and ${V}_{b}^{3}$ as second element mean ${v}_{\overline{b}}^{1 , 1 , 1}$ at (1,1,1); and
calculating a cosine similarity for the first element value at each position in the first image feature and the second element mean at corresponding position, as a second distance value at corresponding position in the second element value distance feature:
   calculating a mean between the first element value ${v}_{a}^{1 , 1 , 1}$ at (1,1,1) in the first image feature and the second element, and calculating a second distance value ${v}_{D 2}^{1 , 1 , 1}$ at (1,1,1) in the second element value distance feature using the cosine similarity:
   ${v}_{D 2}^{1 , 1 , 1} = \frac{{v}_{a}^{1 , 1 , 1} \times {v}_{\overline{b}}^{1 , 1 , 1}}{\sqrt{{\left({v}_{a}^{1 , 1 , 1}\right)}^{2}} \times \sqrt{{\left({v}_{\overline{b}}^{1 , 1 , 1}\right)}^{2}}} .$

This process is repeated likewise for all positions, ultimately yielding *D*₂ ∈ *M* × *N* × *R.*

Calculating the second element value distance feature using the cosine similarity can reflect directional consistency between pixels in a multi-dimensional space, and focus more on semantic similarity.

S102: determining, for the third element value at each position in a current cross feature, whether the third element value at the position requires optimization, based on the first distance value at the corresponding position in the first element value distance feature and the second distance value at the corresponding position in the second element value distance feature. Specifically, whether the third element value at each position requires optimization can be determined by a method as follows:
determining whether the first distance value corresponding to the position is greater than a first target value and whether the second distance value corresponding to the position is less than a second target value, and if the first distance value is greater than the first target value and the second distance value is less than the second target value, determining that the third element value requires optimization.

For a pixel feature at a certain position, if corresponding first distance value is greater than the first target value and the second distance value is less than the second target value, optimization is performed on the third element value, so as to better capture the similarity between the image features.

S103: modifying the third element value, if the third element value at the position requires optimization, based on the second element value at the position in the current second image feature, as a fourth element value at a corresponding position in the corresponding optimized feature.

A product obtained by multiplying the third element value at the corresponding position in the current cross feature by an amplification factor is taken as the fourth element value.

The amplification coefficient herein can be preset, ranging between 0 and 1.

In this way, the element value in the optimized feature can enhance alignment between the cross feature and semantic segmentation elements, so as to obtain a more accurate semantic segmentation result.

S4: fusing, through a decoder, the first image feature, the multiple optimized features, and masks matching each optimized feature, so as to obtain a mask result corresponding to the first image, where the mask result is configured to indicate target pixel regions belonging to the medical semantic label and non-target pixel regions excluded from the medical semantic label in the first image.

In step S4, a TransUnet decoder can be employed, which not only can provide a spatial information preservation capability, but also can enhance global contextual understanding by incorporating Transformer mechanism. The mask result corresponding to the first image output by the decoder can indicate pixel parts belonging to a tumor and pixel portions not belonging to the tumor in the image.

The medical image semantic segmentation method provided in the present invention takes both a segmented image and an unsegmented image of the patient with the same medical semantic label as input to extract features, performs alignment and optimization on the extracted features, so as to further enhance the descriptive effect of certain features for the medical semantic label, and finally decodes to obtain the segmentation result of the medical image of the patient, thus improving the accuracy of the medical semantic segmentation in the rare disease diagnosis and treatment, and providing assistance for diagnosis and treatment of the rare disease patients.

### Embodiment 2

An embodiment of the present invention provides a medical image semantic segmentation mode, specifically obtained through training steps as follows:
acquiring brain MRI images of a same patient taken in different medical visits, designating a latest image as a first image, and remaining images as second images; and acquiring masks corresponding to the first image and each second image, wherein in the masks of the second images, a tumor region can be depicted in white and other regions can be depicted in black;
performing feature extraction on the first image and the second images through a feature extraction network, so as to acquire a first image feature and a corresponding number of second image features;
performing feature cross-alignment between the first image feature and a mean of multiple second image features, so as to obtain multiple cross features; and performing optimization on the multiple cross features, respectively, based on a similarity between the first image feature and the multiple second image features, so as to acquire multiple optimized features;
fusing, through a decoder, the first image feature, the multiple optimized features, and masks matching each optimized feature, so as to obtain a mask result corresponding to the first image, where the mask result is configured to indicate target pixel regions associated with a medical semantic label and non-target pixel regions excluded from the medical semantic label in the first image; and
calculating a loss function based on a deviation between a predicted mask result and an actual mask result of the first image, so as to adjust parameters in the model, and finally generating a trained medical image semantic segmentation model.

### Embodiment 3

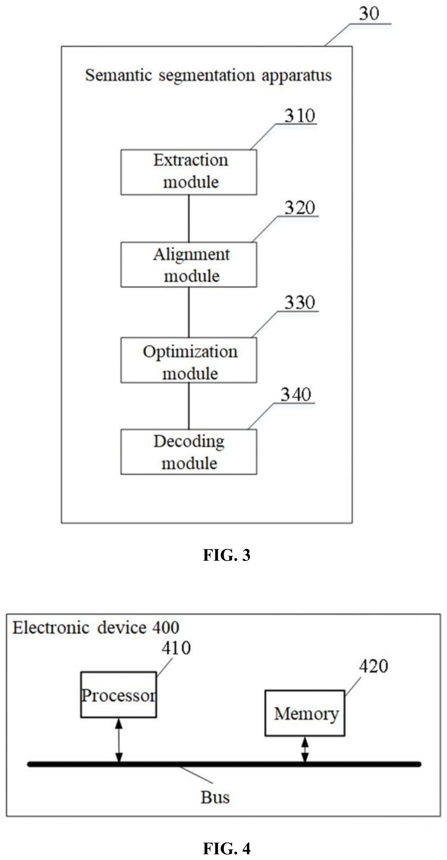

As shown in FIG. 3, based on the same inventive concept, embodiments of the present invention further provide a medical image semantic segmentation apparatus 30, including:
an extraction module 310, configured to extract, through a feature extraction network, a first image feature corresponding to a first image and multiple second image features corresponding to at least one second image, respectively, wherein the at least one second image is image(s) of a target patient taken during historical medical visits and having undergone semantic segmentation for a medical semantic label, and the first image is an image of the target patient currently taken and having pixel regions with the medical semantic label;
an alignment module 320, configured to perform feature cross-alignment between the first image feature and a mean of the multiple second image features, so as to obtain multiple cross features, where a corresponding cross feature for the first image feature and each second image feature is obtained by a method as follows:
   calculating a product, for a first element value at each position in the first image feature, between the first element value and a second element value at a corresponding position in the second image feature, as a third element value at a corresponding position in the corresponding cross feature;
   an optimization module 330, configured to perform optimization on the multiple cross features, respectively, based on a similarity between the first image feature and the multiple second image features, so as to acquire multiple optimized features; and
   a decoding module 340, configured to fuse, through a decoder, the first image feature, the multiple optimized features, and masks matching each optimized feature, so as to obtain a mask result corresponding to the first image, where the mask result is configured to indicate target pixel regions belonging to the medical semantic label and non-target pixel regions excluded from the medical semantic label in the first image.

In a preferred embodiment, the feature extraction network includes a first convolutional block, a second convolutional block, a third convolutional block, a fourth convolutional block, and a fifth convolutional block connected in sequence, wherein each convolutional block includes a first convolutional layer, an asymmetric convolutional layer, and a second convolutional layer connected in sequence.

In a preferred embodiment, for the first image feature and each second image feature, the optimization module 330 performs the optimization on the corresponding cross feature by a following method, so as to acquire corresponding optimized feature:
determining a first element value distance feature based on the first image feature and a current second image feature; determining a second element value distance feature based on the first image feature and remaining second image features; determining, for the third element value at each position in a current cross feature, whether the third element value at the position requires optimization, based on a first distance value at the corresponding position in the first element value distance feature and a second distance value at the corresponding position in the second element value distance feature; and modifying the third element value, if the third element value at the position requires optimization, based on the second element value at the position in the current second image feature, as a fourth element value at a corresponding position in the corresponding optimized feature.

In a preferred embodiment, the optimization module 330 determines whether the third element value at each position requires optimization by a method as follows:
determining whether the first distance value corresponding to the position is greater than a first target value and whether the second distance value corresponding to the position is less than a second target value, and if the first distance value is greater than the first target value and the second distance value is less than the second target value, determining that the third element value requires optimization.

In a preferred embodiment, the optimization module 330 calculates the fourth element value by a method as follows:
multiplying the third element value at the corresponding position in the current cross feature by an amplification factor to obtain a product as the fourth element value.

In a preferred embodiment, the optimization module 330 calculates the first element value distance feature by a method as follows:
performing decomposition on the first element value at each position in the first image feature and the second element value at a corresponding position in the current second image feature, so as to acquire a first integer element value and a first decimal element value corresponding to the first element value, and a second integer element value and a second decimal element value corresponding to the second element value, respectively; calculating a first difference between the first integer element value and the second integer element value; calculating a second difference between the first decimal element value and the second decimal element value; and calculating a sum of an absolute value of the first difference and an absolute value of the second difference as a first distance value at a corresponding position in the first element value distance feature.

In a preferred embodiment, the optimization module 330 calculates the second element value distance feature by a method as follows:
for second element values at corresponding position in the remaining second image features, calculating a second element mean at the position; and calculating a cosine similarity for the first element value at each position in the first image feature and the second element mean at a corresponding position, as a second distance value at corresponding position in the second element value distance feature.

With reference to FIG. 4, FIG. 4 is a structural schematic diagram of an electronic device provided in embodiments of the present invention. As shown in FIG. 4, the electronic device 400 includes a processor 410, a memory 420, and a bus 430.

The memory 402 stores machine-readable instructions executable by the processor 401. When the electronic device 400 is running, the processor 401 and the memory 402 communicate via the bus 430. The machine-readable instructions, when executed by the processor 401, can execute the steps of the medical image semantic segmentation method in the method embodiments in FIG. 1. Reference can be made to the method embodiment for specific implementation, which will not be elaborated herein.

Embodiments of the present invention further provide a computer-readable storage medium, on which a computer program is stored. The computer program, when run by a processor, executes the steps of the medical image semantic segmentation method in the method embodiments in FIG. 1. Reference can be made to the method embodiment for specific implementation, which will not be elaborated herein.

It would be clear to those skilled in the field that, for the convenience and conciseness of the description, reference can be made to corresponding processes in the preceding method embodiments for specific working processes of system, apparatus, and unit described in the above, which will not be elaborated herein.

In the embodiments provided in the present invention, it should be understood that the disclosed apparatus and method may be implemented in other manners. The apparatus embodiments described in the above are merely exemplary. For example, units are merely divided according to logical functions, but they may be divided in other ways in practical implementation. For another example, a plurality of units or components can be combined or integrated into another system, or some features can be omitted, or not executed. In addition, a mutual coupling, direct coupling or communication connection shown or discussed can be an indirect coupling or communication connection through some communication interfaces, devices or units, and can be in an electrical form, a mechanical form or other forms.

In addition, units described as separate components may be or may not be physically separated. The components shown as units may be or may not be physical units, i.e., they may be located at one place or distributed onto multiple network units. Some or all of the units can be selected as actually needed to achieve the objective of the solutions in the embodiments.

Furthermore, individual functional modules in each embodiment of the present invention can be integrated together to form an independent part, or each module may exist alone, or two or more modules may be integrated to form an independent part.

It should be noted that functionality, if implemented in the form of software functional unit and sold or used as standalone product, can be stored in a computer-readable storage medium. Based on such understanding, the technical solutions of the present invention in essence or parts making contribution to the prior art or parts of the technical solutions can be embodied in form of a software product. This computer software product is stored in a storage medium, and includes several instructions for causing a computer device (which may be a personal computer, a server or a network device, etc.) to execute all or some of the steps of the method of various embodiments of the present invention. The aforementioned storage medium includes various media capable of storing program code, such as USB flash drives, portable hard drives, read-only memory (ROM), random access memory (RAM), magnetic disks or optical disks.

Herein, the relational terms such as first and second are used only to distinguish one entity or operation from another, rather than necessarily requiring or implying any such actual relationship or sequence between these entities or operations.

The above-mentioned are merely for the embodiments of the present invention and not intended to limit the scope of protection of the present invention. For those skilled in the art, various modifications and changes could be made to the present invention. Any modifications, equivalent substitutions, improvements and so on made within the spirit and principle of the present invention should be covered within the scope of protection of the present invention.

## Claims

1. A medical image semantic segmentation method, **characterized by** comprising steps of:
extracting, through a feature extraction network, a first image feature corresponding to a first image and second image features corresponding to multiple second images, respectively, wherein the at least one second image is images of a target patient taken during historical medical visits and having undergone semantic segmentation for a medical semantic label, and the first image is an image of the target patient currently taken and having pixel regions with the medical semantic label;
performing feature cross-alignment between the first image feature and the multiple second image features, so as to obtain multiple cross features;
performing optimization on a corresponding cross feature by a following method, for the first image feature and each second image feature, so as to acquire a corresponding optimized feature: determining a first element value distance feature based on the first image feature and a current second image feature; determining a second element value distance feature based on the first image feature and remaining second image features; determining, for the third element value at each position in a current cross feature, whether the third element value at the position requires optimization, based on a first distance value at the corresponding position in the first element value distance feature and a second distance value at the corresponding position in the second element value distance feature; and modifying the third element value, if the third element value at the position requires optimization, based on the second element value at the position in the current second image feature, as a fourth element value at a corresponding position in the corresponding optimized feature;
fusing, through a decoder, the first image feature, the multiple optimized features, and masks matching each optimized feature, so as to obtain a mask result corresponding to the first image, wherein the mask result is configured to indicate target pixel regions belonging to the medical semantic label and non-target pixel regions excluded from the medical semantic label in the first image, wherein
a corresponding cross feature for the first image feature and each second image feature is obtained by a method as follows:
calculating a product, for a first element value at each position in the first image feature, between the first element value and a second element value at a corresponding position in the second image feature, as a third element value at a corresponding position in the corresponding cross feature.

2. The method according to claim 1, wherein the feature extraction network comprises a first convolutional block, a second convolutional block, a third convolutional block, a fourth convolutional block, and a fifth convolutional block connected in sequence, wherein each convolutional block includes a first convolutional layer, an asymmetric convolutional layer, and a second convolutional layer connected in sequence.

3. The method according to claim 1, wherein whether the third element value at each position requires optimization is determined by a method as follows:
determining whether the first distance value corresponding to the position is greater than a first target value and whether the second distance value corresponding to the position is less than a second target value, and
if the first distance value is greater than the first target value and the second distance value is less than the second target value,
determining that the third element value requires optimization.

4. The method according to claim 1, wherein the fourth element value is calculated by a method as follows:
multiplying the third element value at corresponding position in the current cross feature by an amplification factor to obtain a product as the fourth element value.

5. The method according to claim 1, wherein the first element value distance feature is determined by a method as follows:
performing decomposition on the first element value at each position in the first image feature and the second element value at a corresponding position in the current second image feature, so as to acquire a first integer element value and a first decimal element value corresponding to the first element value, and a second integer element value and a second decimal element value corresponding to the second element value, respectively;
calculating a first difference between the first integer element value and the second integer element value;
calculating a second difference between the first decimal element value and the second decimal element value; and
calculating a sum of an absolute value of the first difference and an absolute value of the second difference as a first distance value at a corresponding position in the first element value distance feature.

6. The method according to claim 1, wherein the second element value distance feature is determined by a method as follows:
calculating, for second element values at corresponding position in the remaining second image features, a second element mean at the position; and
calculating a cosine similarity for the first element value at each position in the first image feature and the second element mean at a corresponding position, as a second distance value at corresponding position in the second element value distance feature.

7. The method according to claim 2, wherein the first convolutional block has a convolutional kernel of 3×3×3, the second convolutional block has a convolutional kernel of 3×1×1, the third convolutional block has a convolutional kernel of 1×3×1, the fourth convolutional block has a convolutional kernel of 1×1×3, and the fifth convolutional block has a convolutional kernel of 1×1×1.

8. The method according to claim 1, wherein the feature cross-alignment between the first image feature and the mean of the multiple second image features is performed by using Hadamard Product.

9. The method according to claim 1, wherein Manhattan distance is utilized to calculate a pixel-wise similarity between the first image feature and the second image feature.

10. The method according to claim 4, wherein the amplification factor ranges between 0 and 1.

11. The method according to claim 1, wherein the decoder is a TransUnet decoder.

12. A medical image semantic segmentation apparatus, **characterized by** comprising:
an extraction module, configured to extract, through a feature extraction network, a first image feature corresponding to a first image and multiple second image features corresponding to multiple second images, respectively, wherein the multiple second images are images of a target patient taken during historical medical visits and having undergone semantic segmentation for a medical semantic label, and the first image is an image of the target patient currently taken and having pixel regions with the medical semantic label;
an alignment module, configured to perform feature cross-alignment between the first image feature and the multiple second image features, so as to obtain multiple cross features, wherein a corresponding cross feature for the first image feature and each second image feature is obtained by a method as follows:
calculating a product, for a first element value at each position in the first image feature, between the first element value and a second element value at a corresponding position in the second image feature, as a third element value at a corresponding position in the corresponding cross feature;
an optimization module, configured to perform optimization on a corresponding cross feature by a following method, for the first image feature and each second image feature, so as to acquire a corresponding optimized feature: determining a first element value distance feature based on the first image feature and a current second image feature; determining a second element value distance feature based on the first image feature and remaining second image features; determining, for the third element value at each position in a current cross feature, whether the third element value at the position requires optimization, based on a first distance value at the corresponding position in the first element value distance feature and a second distance value at the corresponding position in the second element value distance feature; and modifying the third element value, if the third element value at the position requires optimization, based on the second element value at the position in the current second image feature, as a fourth element value at a corresponding position in the corresponding optimized feature; and
a decoding module, configured to fuse, through a decoder, the first image feature, the multiple optimized features, and masks matching each optimized feature, so as to obtain a mask result corresponding to the first image, wherein the mask result is configured to indicate target pixel regions belonging to the medical semantic label and non-target pixel regions excluded from the medical semantic label in the first image.

13. An electronic device, **characterized by** comprising: a processor, a memory, and a bus, wherein the memory stores machine-readable instructions executable by the processor, when the electronic device is running, the processor and the memory communicate via the bus, and the machine-readable instructions, when executed by the processor, executes the steps of the medical image semantic segmentation method according to any one of claims 1 to 7.

14. A computer-readable storage medium, **characterized in that** a computer program is stored on the computer-readable storage medium, and the computer program, when run by a processor, executes the steps of the medical image semantic segmentation method according to any one of claims 1 to 7.
